# FASCICULE DE BREVET EUROPEEN

(11) **EP 2 198 825 B1**
(45) Date de publication et mention de la délivrance du brevet: **24.01.2018**
(21) Numéro de dépôt: 09178880.2
(22) Date de dépôt: 11.12.2009
(51) Int. Cl.: A61K 8/02, A61K 8/06, A61K 8/60, A61Q 19/00, A61Q 5/00, A61K 8/46, A61K 8/86

(54) **Emulsion huile dans eau présentant un pH allant de 3 à 5**
Öl in Wasseremulsion mit einem pH-Wert von 3 bis 5
Oil in water emulsion having a pH comprised between 3 and 5

(30) Priorité: 18.12.2008 FR 0858755
(43) Date de publication de la demande: 23.06.2010
(73) Titulaire: L'Oréal, 75008 Paris (FR)
(72) Inventeur: L'Alloret, Florence, 75014, Paris (FR); Simonnet, Jean-Thierry, 94240, Cachan (FR)
(74) Mandataire: Prevel, Estelle Nicole

(56) Documents cités:
- EP-A- 0 641 557
- EP-A- 0 705 593
- FR-A- 2 718 021

## Description

La présente invention concerne une composition cosmétique ou dermatologique sous forme d'émulsion du type huile dans eau. Elle concerne plus particulièrement une composition sous forme d'émulsion huile dans eau formée par des globules huileux pourvus d'un enrobage cristal liquide lamellaire, dispersés dans une phase aqueuse. L'invention concerne également un procédé de préparation d'une telle composition et son application au traitement de la peau et de la matière kératinique.

De nombreuses compositions cosmétiques pour la peau sous forme d'émulsion présentent un pH supérieur à 6, principalement pour des raisons de stabilité de formulation. En effet, la stabilité des émulsions vis-à-vis de la coalescence est souvent renforcée à l'aide de tensio-actifs ioniques qui doivent être formulés à un pH au moins égal à 6 pour être sous forme ionisée.
On connaît par exemple du document EP0641557 des émulsions huile dans eau formée de globules huileux de taille définie pourvus d'un enrobage cristal liquide lamellaire et comprenant un agent tensioactif lipophile et un agent tensioactif hydrophile. Ces émulsions comprennent un acide gras pour améliorer leur stabilité et cet acide, du fait de son faible pKa, doit être formulé à un pH au moins égal à 6 pour être sous forme ionisée.
Le document EP0705593 décrit des émulsions huile dans eau formée de globules huileux de taille définie pourvus d'un enrobage cristal liquide lamellaire et comprenant un agent tensioactif lipophile, un agent tensioactif hydrophile et un composé amphiphile ionique conférant à l'émulsion un pH allant de 5,5 à 7,5. Ces composés amphiphiles ne permettent pas l'obtention d'émulsion à bas pH.
Par ailleurs, de nombreux gélifiants assurant la stabilité des émulsions vis-à-vis du crémage ou de la sédimentation voient leurs propriétés de gélification fortement limitées à un pH inférieur à 6.
Or on cherche à formuler des émulsions dont le pH est inférieur à 6, notamment pour des raisons d'innocuité car un tel pH est plus proche du pH de la peau, lui-même compris entre 4,5 et 6, et la protection microbiologique de formulations présentant cette gamme de pH est possible avec de plus faibles taux de conservateurs.
En outre les compositions présentant cette gamme de pH permettent de formuler plus aisément les actifs hydrosolubles sous forme acide, tels que l'acide salicylique ou les hydroxyacides..

Il apparaît ainsi nécessaire de disposer d'émulsions cosmétiques qui sont stables à un pH allant de 3 à 5 et présentant une large gamme de textures allant du fluide à la crème.
La demanderesse a découvert qu'il était possible de réaliser ces objectifs en proposant une composition cosmétique sous forme d'émulsion comprenant un système tensiocatif particulier.
Plus précisément la présente invention a pour objet une composition cosmétique sous forme d'émulsion huile dans eau formée de globules huileux pourvus chacun d'un enrobage cristal liquide lamellaire et dispersés dans une phase aqueuse, ladite composition comprenant au moins un agent tensioactif lipophile, au moins un agent tensioactif hydrophile et d'au moins un composé amphiphile de nature ionique à un pH allant de 3 à 5. Chaque globule huileux de la composition selon l'invention est unitairement enrobé d'une couche monolamellaire et d'au moins une bicouche lamellaire obtenues à partir de l'agent tensioactif lipophile, de l'agent tensioactif hydrophile et du composé amphiphile ionique.
L'association des tensioactifs et du composé amphiphile spécifique permet de réaliser des émulsions présentant un pH faible, tout en étant stables et de texture fluide.
L'invention permet de disposer d'émulsions particulièrement stables, ayant des gouttelettes de phase grasse de petite taille, revêtues d'une couche mono et d'une couche oligo lamellaire extrêmement fine. Ainsi l'utilisation d'agents gélifiants, si nécessaire, peut être faite en des taux réduits.
On entend par couche oligolamellaire une couche comprenant de 2 à 5 lamelles lipidiques (on entend par lamelle lipidique une membrane de type bicouche lipidique telle que décrites dans le domaine des liposomes par exemple).

La taille moyenne des globules huileux revêtus peut être notamment inférieure à 1 micromètre, de préférence à 800. 10⁻³ microns.
Dans la présente invention on entend par taille moyenne des globules huileux, la taille moyenne en nombre des gouttes, telle que mesurée par exemple à l'aide d'un analyseur de taille BI-90 (Brookhaven instrument).

Par « émulsions stables », on entend des émulsions qui après 2 mois de stockage à toutes températures comprises entre 4°C et 50°C ne présentent aucun changement macroscopique de couleur, d'odeur, de viscosité, ni de variation de pH ainsi qu'aucune variation d'aspect microscopique.

La viscosité des compositions obtenues peut aller de très fluide (spray) à très visqueuse (crème). La composition de l'invention présente de préférence une viscosité supérieure ou égale à 0,1 Pa.s, pouvant aller par exemple de 0,1 Pa.s à 100 Pa.s, de préférence de 0,1 Pa.s à 10 Pa.s à une température de 25°C, la viscosité étant mesurée à l'aide d'un Rhéomat 180 (Société LAMY), équipé d'un mobile MS-R1, MS-R2, MS-R3, MS-R4 ou MS-R5 choisi en fonction de la consistance de la composition, tournant à un vitesse de rotation de 200 t.min-1. Les compositions cosmétiques selon l'invention présentent un pH allant de 3 à 5 et mieux de 4 à 5. L'invention a également pour objet un procédé de traitement cosmétique de la peau, des cheveux, et/ou des lèvres, caractérisé en ce qu'on applique sur la peau, les cheveux et/ou les lèvres, une composition cosmétique telle que définie plus haut.
Les tensioactifs lipophile et hydrophiles utilisés dans la présente invention sont non ioniques.

### Tensioactif lipophile

Par tensioactif lipophile, on entend un tensio-actif dont le paramètre HLB va de 2 à 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.
La valeur HLB selon GRIFFIN est définie dans J. Soc. Cosm. Chem. 1954 (volume 5), pages 249-256.

Les tensioactifs lipophiles de HLB allant de 2 à 5 sont choisis parmi : les mono, di ou triesters de sucrose et d'acides gras comprenant de 12 à 30 atomes de carbone, de préférence de 14 à 20 atomes de carbone tel que l'acide stéarique, en particulier le tristérarate de sucrose, le distéarate de sucrose et leurs mélanges.

L'agent tensioactif lipophile peut être présent en une teneur allant de 0,1 à 15% en poids par rapport au poids total de la composition, de préférence de 0,5 à 5% en poids.

### Tensioactif hydrophile

L'agent tensioactif hydrophile présente un HLB allant de 8 à 12.

Les tensioactifs hydrophiles de HLB allant de 8 à 12 sont choisis parmi :
- les esters d'acide gras (notamment d'acide en C12-C30, et de préférence en C12-C20) et d'éthers de sorbitol et/ou de ou de sorbitan, oxyéthylénés (pouvant comporter de 2 à 30 groupes oxyéthylénés), tels que les esters d'acide stéarique et de sorbitol et/ou de sorbitan comprenant de 2 à 20 groupes OE comme le polysorbate-60, le polysorbate-61, le sorbeth 3 isosteratate, le monostéarate de sorbitan polyoxyéthyléné 4 OE, le tristéarate de sorbitan polyoxyéthyléné 20 OE. De préférence le tensioactif hydrophile est choisi parmi le monostéarate de sorbitane polyoxyéthyléné 20 OE, le monostéarate de sorbitane polyoxyéthyléné 4 OE, le tristéarate de sorbitan polyoxyéthyléné 20 OE, le distéarate de méthylglucose polyoxyéthyléné 20 OE, le monostéarate polyoxyéthyléné 8 OE, le monostéarate polyoxyéthyléné 10 OE, le distéarate polyoxyéthyléné 12 OE, le monostéarate d'hexaglycéryl et leurs mélanges, encore plus partculièrement parmi le monostéarate de sorbitane polyoxyéthyléné 20 OE, le monostéarate de sorbitane polyoxyéthyléné 4 OE ou un mélange. L'agent tensioactif hydrophile peut être présent en une teneur allant de 0,05 à 15% en poids, de préférence de 0,1 à 10% en poids par rapport au poids total de la composition, de préférence de 0,5 à 5% en poids. Composé amphiphile de nature ionique à un pH allant de 3 à 5 Par composé amphiphile de nature ionique à un pH allant de 3 à 5, on entend un composé comportant une partie lipophile et une partie hydrophile, qui est de nature ionique et dont la charge existe dans ce domaine de pH à l'état non neutralisé. On entend plus précisément un composé dont au moins 80% des groupes ionisables sont sous forme d'ions à un pH allant de 3 à 5. On utilise un composé amphiphile de nature ionique à un pH allant de 3 à 5 et mieux de 4 à 5. De tels composés amphiphiles ioniques sont choisis parmi :
- les sels de sodium et de potassium du monocétyle phosphate, comme par exemples le composé commercialisé sous la référence Amphisol K par la société DSM.

Le composé amphiphile ionique peut être présent en une teneur allant de 0,05 à 10% en poids par rapport au poids total de la composition, de préférence de 0,5 à 5% en poids.

L'enrobage selon l'invention des globules huileux demande de préférence l'utilisation d'une quantité totale d'agent tensioactif hydrophile, d'agent tensioactif lipophile et de composé amphiphile ionique allant de 2% à 6%, de préférence de 3 à 5% en poids par rapport au poids total de la composition.

Les quantités relatives de tensioactifs lipophile, hydrophile et de composé amphiphile ionique vont de préférence respectivement de 35 à 55% en poids, de 25 à 40% en poids et de 15 à 35% en poids par rapport au poids total de l'ensemble des tensioactifs lipophile, hydrophile et du composé amphiphile ionique.

Selon un mode de réalisation, la composition selon l'invention est exempte de composés amphiphiles de nature ionique à un pH supérieur à 5,5, c'est-à-dire de composés qui lorsqu'ils sont dispersés dans l'eau sans être neutralisés se trouvent sous forme ionique à ce pH.
Selon un mode de réalisation, la composition selon l'invention est exempte d'acide gras, en particulier d'acide stéarique..

### Phase aqueuse

La phase aqueuse de la composition selon l'invention comprend de l'eau et éventuellement un ou plusieurs composés miscibles à l'eau ou au moins en partie miscibles à l'eau, comme les polyols ; les mono-alcools inférieurs en C₂ à C₈, tels que l'éthanol et l'isopropanol. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple les glycols comme le butylène glycol, le propylène glycol, l'isoprène glycol, le glycérol et les polyéthylène glycols comme le PEG-8, le sorbitol, les sucres comme le glucose.

La phase aqueuse peut comprendre aussi tout additif habituel hydrosoluble ou hydrodispersible comme indiqué ci-après.

La phase aqueuse peut représenter de 60 à 98 % en poids, de préférence de 65 à 95 % en poids, mieux de 70 à 90 % en poids, et encore mieux de 70 à 85 % en poids par rapport au poids total de la composition.
Cette quantité de phase aqueuse ne comprend pas la quantité de tensioactif ni de gélifiant hydrophile.

### Agents gélifiants

Comme exposé plus haut, la viscosité des compositions de l'invention peut être ajustée à l'aide de gélifiant(s) hydrophile(s) présents dans la phase continue de l'émulsion. Des exemples de gélifiants hydrophiles sont
- les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Goodrich ;
- les polyacrylamides et les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination « Hostacerin AMPS » (nom INCI : ammonium polyacryldimethyltaurate) ; les copolymères anioniques réticulés d'acrylamide et d'AMPS, se présentant sous la forme d'une émulsion, tels ceux commercialisés sous le nom de SEPIGEL 305 (nom C.T.F.A. : Polyacrylamide / C13-14 Isoparaffin / Laureth-7), sous le nom de SIMULGEL 600 (nom C.T.F.A. : Acrylamide / Sodium acryloyldimethyltaurate copolymer / Isohexadecane / Polysorbate 80) par la société SEPPIC, sous la dénomination de Simulgel EG (nom C.T.F.A : Sodium acrylate/Sodium acryloyldimethyl taurate copolymer and isohexadecane and polysorbate 80) ;
- les copolymères acrylate/acrylonitrile tels que le HYPAN SS201 commercialisé par la société Kingston ;
- les biopolymères polysaccharidiques comme la gomme de xanthane, la gomme de guar, les alginates, les celluloses modifiées ou non ;
- les composés inorganiques tels que les smectites, les hectorites modifiées ou non telles que les produits BENTONE commercialisés par la société Rheox, les produits LAPONITE commercialisés par la société Southern Clay Products, le produit VEEGUM HS commercialisé par la société R.T.Vanderbilt ;
- et leurs mélanges.

On utilise de préférence comme gélifiant hydrophile les polymères et copolymères d'acide 2-acrylamido 2-méthylpropane sulfonique, éventuellement réticulés .

Le gélifiant hydrophile peut être présent dans la composition en une teneur en matières sèches allant de 0,05% à 5% en poids, de préférence de 0,1% à 3% en poids, et encore mieux de 0,3% et 2% en poids par rapport au poids total de la composition.
Selon un mode de réalisation, le gélifiant hydrophile est présent dans la composition en une teneur en matières sèches inférieure ou égale à 2% en poids, de préférence inférieure ou égale à 1% en poids par rapport au poids total de la composition.

Cette concentration est ajustée afin que la viscosité de la composition mesurée à l'aide d'un viscosimètre Rhéomat 180 à 25°C pour une vitesse de rotation de 200 RPM soit avantageusement supérieure ou égale à 0,10 Pa.s.

### Phase huileuse

La phase huileuse est une phase grasse comprenant au moins un corps gras choisi parmi les corps gras liquides à température ambiante (20-25°C) ou huiles, volatiles ou non, d'origine végétale, minérale ou synthétique, et leurs mélanges. Ces huiles sont physiologiquement acceptables.

La phase huileuse peut comprendre aussi tout additif habituel liposoluble ou lipodispersible comme indiqué ci-après.

Elle peut notamment comprendre d'autres corps gras tels que des cires, des composés pâteux, des alcools gras, des acides gras. La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les alcanes linéaires volatils, avantageusement d'origine végétale, comprenant de 7 à 17 atomes de carbone, en particulier de 9 à 15 atomes de carbone, et plus particulièrement de 11 à 13 atomes de carbone.
A titre d'exemples d'alcanes linéaires volatils convenant à l'invention, on peut mentionner ceux décrits dans la demande de brevet de la société Cognis WO 2007/068371.
A titre d'exemple d'alcane linéaire volatil convenant à l'invention, on peut citer le n-nonane (C₉), le n-décane (C₁₀), le n-undécane (C₁₁), le n-dodécane (C₁₂), le n-tridécane (C₁₃), le n-tétradecane (C₁₄), le n-pentadécane (C₁₅), le n-héxadécane (C₁₆) et le
n-heptadécane (C₁₇) et leurs mélanges. Selon une forme particulièrement préférée, on utilisera un mélange d'undécane (C₁₁) et de tridécane (C₁₃) comme le produit commercialisé sous la référence de CETIOL UT par la Société Cognis,
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode de réalisation, la composition de l'invention comprend au moins une huile choisie parmi les huiles de silicone, les hydrocarbures linéaires ou ramifiés, les éthers et les esters de synthèse, et leurs mélanges, et notamment choisie parmi les huiles de silicone volatiles et les hydrocarbures ramifiés comme l'huile de Parléam®, et leurs mélanges.

La quantité de phase huileuse dans la composition de l'invention peut aller de 2 à 50% en poids par rapport au poids total de la composition, de 5 à 30% en poids et mieux de 7 à 25 % en poids par rapport au poids total de la composition.
Cette quantité de phase huileuse ne comprend pas la quantité de tensioactif.

En particulier la composition selon l'invention comprend de 2 à 50% en poids d'huiles par rapport au poids total de la composition, de préférence de 5 à 30% en poids d'huiles et mieux de 7 à 25 % en poids par rapport au poids total de la composition.

Selon un mode de réalisation particulier, la phase huileuse de la composition selon l'invention comprend au moins une huile ester choisie parmi les dérivés lipophiles d'acides aminés, tels que le lauroyl sarcosinate d'isopropyle, les esters d'acide gras, linéaires ou ramifiés, comprenant de 8 à 29 atomes de carbone et d'alcools, linéaires ou ramifiés, de préférence d'alcool ramifiés, comprenant de 3 à 15 atomes de carbone, tels que le myristate d'ispropropyle, l'isononanoate d'isononyle et leurs mélanges.
De telles huiles permettent encore d'améliorer la stabilité de la composition.

Selon un mode de réalisation particulier, la phase huileuse de la composition selon l'invention comprend au moins 25% en poids d'au moins une huile ester choisie parmi les dérivés lipophiles d'acides aminés, tels que le lauroyl sarcosinate d'isopropyle, les esters d'acide gras, linéaires ou ramifiés, comprenant de 8 à 29 atomes de carbone et d'alcools, linéaires ou ramifiés, , de préférence d'alcool ramifiés, comprenant de 3 à 15 atomes de carbone, tels que le myristate d'ispropropyle, l'isononanoate d'isononyle et leurs mélanges.
En particulier les huiles ester ci-dessus représentent au moins 25% en poids du poids total des huiles présentes dans la composition.

### Additifs

De façon connue, la composition pour application topique de l'invention peut contenir également un ou plusieurs des adjuvants habituels dans le domaine cosmétique ou dermatologique. Comme adjuvants, on peut citer par les gélifiants, les actifs, les conservateurs, les antioxydants, les parfums, les solvants, les sels, les charges, les filtres solaires (= filtres U.V.), les matières colorantes, et encore les vésicules lipidiques ou tout autre type de vecteur (microcapsules par exemple), et leurs mélanges. Ces adjuvants sont utilisés dans les proportions habituelles dans le domaine cosmétique, et par exemple de 0,01 à 30 % du poids total de la composition, et ils sont, selon leur nature, introduits dans la phase aqueuse de la composition ou dans la phase huileuse, ou encore dans des vésicules ou tout autre type de vecteur. Ces adjuvants ainsi que leurs concentrations doivent être tels qu'ils ne modifient pas la propriété recherchée pour l'émulsion de l'invention, en particulier le pH de la composition.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphériques organiques, les fibres ; et leurs mélanges. Comme poudres sphériques organiques, on peut citer par exemple les poudres de polyamide et notamment les poudres de Nylon® telles que Nylon-1 ou Polyamide 12, commercialisées sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle commercialisées par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges. Ces charges peuvent être présentes dans des quantités allant de 0 à 20 % en poids et de préférence de 0,5 à 10 % en poids par rapport au poids total de la composition.

Comme actifs utilisables dans la composition de l'invention, on peut citer par exemple, les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine K, la vitamine C (acide ascorbique), la vitamine B5 (panthénol), la vitamine B3 ou PP (niacinamide), les dérivés de ces vitamines (notamment esters) et leurs mélanges ; les agents kératolytiques et/ou desquamants tels que l'acide salicylique et ses dérivés, les alpha-hydroxyacides comme l'acide lactique et l'acide glycolique et leurs dérivés, l'acide ascorbique et ses dérivés ; l'urée ; la caféine ; les dépigmentants tels que l'acide kojique, l'hydroquinone et l'acide caféique ; l'acide salicylique et ses dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les enzymes ; les flavonoïdes ; les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les céramides ; les agents anti-inflammatoires ; les agents apaisants ; les agents matifiants ; les agents anti-chute et/ou repousse des cheveux ; les agents anti-rides ; les huiles essentielles ; et leurs mélanges ; et tout actif approprié pour le but final de la composition.

Les filtres U.V. peuvent être organiques ou minéraux (ou filtres U.V. physiques). Ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples d'agents photoprotecteurs ou filtres organiques on peut citer ceux désignés ci-dessous sous leur nom INCI :

### Dérivés de dibenzoylméthane:

Butylmethoxydibenzoylmethane vendu notamment sous le nom commercial PARSOL MCX par DSM Nutritional Products, Inc,,

### Dérivés cinnamiques :

Ethylhexyl Methoxycinnamate vendu notamment sous le nom commercial PARSOL MCX par DSM Nutritional Products, Inc,,
Isopropyl Methoxycinnamate
Isoamyl Methoxycinnamate vendu sous le nom commercial NEO HELIOPAN E 1000 par SYMRISE,
DEA Methoxycinnamate,
Diisopropyl Methylcinnamate,
Glyceryl Ethylhexanoate Dimethoxycinnamate

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par SYMRISE,
Dipropyleneglycol Salicylate vendu sous le nom « DIPSAL » par SCHER,
TEA Salicylate, vendu sous le nom « NEO HELIOPAN TS » par SYMRISE,

### Dérivés de béta,béta-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle vendu sous le nom commercial « UVINUL A+ » par BASF.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial « NEO HELIOPAN AP » par SYMRISE,

### Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,

### Dérivés de triazine :

- Bis-Ethylhexyloxyphenol Methoxyphenyl Triazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris(4'-amino benzalmalonate de dinéopentyle)-s-triazine
- la 2,4,6-tris-(4'-amino benzalmalonate de diisobutyle)-s-triazine,
- la 2,4bis (4'-aminobenzoate de n-butyle)-6-(aminopropyltrisiloxane)-s-triazine,
- la 2,4bis (4'-aminobenzalmalonate de dinéopentyle)-6-(4'-aminobenzoate de n-butyle)-s-triazine,
- les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.

### Dérivés anthraniliques :

Menthyl anthranilate vendu sous le nom commercial commercial « NEO HELIOPAN MA » par SYMRISE,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par DSM Nutritional Products, Inc

### Dérivés de 4,4-diarylbutadiène :

-1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

### Dérivés de mérocyanine

Octyl-5-N,N-diethylamino-2-phenysulfonyl-2,4-pentadienoate

Les filtres organiques préférentiels sont choisis parmi Butylmethoxydibenzoylmethane
Ethylhexyl Methoxycinnamate
Ethylhexyl Salicylate,
Homosalate,
Octocrylene,
Phenylbenzimidazole Sulfonic Acid,
Benzophenone-3,
2-(4-diethylamino-2-hydroxybenzoyl)-benzoate de n-hexyle.
Terephthalylidene Dicamphor Sulfonic Acid,
Methylène bis-Benzotriazolyl Tetramethylbutylphénol
Bis-Ethylhexyloxyphenol Methoxyphenyl Triazine
Ethylhexyl triazone,
Diethylhexyl Butamido Triazone,
la 2,4,6-tris(4'-amino benzalmalonate de dinéopentyle)-s-triazine
la 2,4,6-tris-(4'-amino benzalmalonate de diisobutyle)-s-triazine
la 2,4bis (4'-aminobenzoate de n-butyle)-6-(aminopropyltrisiloxane)-s-triazine,
la 2,4bis (4'-aminobenzalmalonate de dinéopentyle)-6-(4'-aminobenzoate de n-butyle)-s-triazine,
Drometrizole Trisiloxane
Octyl-5-N,N-diethylamino-2-phenysulfonyl-2,4-pentadienoate et leurs mélanges.

Les filtres inorganiques peuvent être choisis parmi des pigments d'oxydes métalliques enrobés ou non dont la taille moyenne des particules primaires: est préférentiellement comprise entre 5.10-3 µm et 100. 10⁻³ µm (de préférence entre 10. 10⁻³ µm et 50. 10-3 µm) comme par exemple des pigments d'oxyde de titane (amorphe ou cristallisé sous forme rutile et/ou anatase), de fer, de zinc, de zirconium ou de cérium ou leurs mélanges qui sont tous des agents photoprotecteurs UV bien connus en soi.
Les pigments peuvent être enrobés ou non enrobés.
Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :
- de silice tels que le produit "SUNVEIL" de la société IKEDA et le produit "Eusolex T-AVO" de la société MERCK
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 TV, MT 100 TX, MT 100 Z, MT-01 de la société TAYCA, les produits "Solaveil CT-10 W", "Solaveil CT 100" et "Solaveil CT 200" de la société UNIQEMA,
- de silice, d'alumine et d'acide alginique tel que le produit " MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA, ou le produit SMT-100 WRS de la société TAYCA.
- d'alumine et traités par une silicone tels que les produits "TIPAQUE TTO-55 (S)" de la société ISHIHARA, ou "UV TITAN M 262" de la société KEMIRA,
   de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO2 traité par l'octyl triméthyl silane et dont la taille moyenne des particules élémentaires est comprise entre 25.10⁻³ et 40. 10⁻³ µm tel que celui vendu sous la dénomination commerciale "T 805" par la société DEGUSSA SILICES, le TiO2 traité par un polydiméthylsiloxane et dont la taille moyenne des particules élémentaires est de 21. 10⁻³ µm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25. 10⁻³ µm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".
Les pigments d'oxyde de zinc enrobés sont par exemple
- ceux commercialisés sous la dénomination « Z-COTE HP1 » par la société SUNSMART (ZnO enrobé dimethicone) ;
- ceux commercialisés sous la dénomination "Oxide zinc CS-5" par la société Toshibi (ZnO enrobé par polymethylhydrogenesiloxane) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% d'oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane).

Les pigments d'oxyde de cérium non enrobé sont vendus par exemple sous la dénomination "COLLOIDAL CERIUM OXIDE" par la société RHONE POULENC.
Des pigments d'oxyde de fer non enrobés de taille de l'ordre de 10⁻³ microns sont par exemple vendus par la société ARNAUD ou par la société MITSUBISHI sous la dénomination "TY-220".

Les pigments d'oxyde de fer enrobés sont par exemple vendus par la société ARNAUD sous les dénominations ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

La quantité totale de filtres UV organiques dans les compositions selon l'invention peut aller par exemple de 0,1 à 20% en poids par rapport au poids total de la composition, et de préférence allant de 0,2 à 10% en poids par rapport au poids total de la composition.

Comme filtres physiques pouvant être ajoutés dans la composition de l'invention, on peut citer par exemple les pigments d'oxydes métalliques, enrobés ou non enrobés, notamment les oxydes de titane, de fer, de zirconium, de zinc ou de cérium, et leurs mélanges, ces oxydes pouvant être sous forme de particules de taille micronique ou de l'odre de 10⁻³ microns, éventuellement enrobées.

Les compositions se présentent le plus fréquemment sous forme de spray, lait, crème ou gel, d'autres modes de présentation n'étant pas exclus. Elles sont transparentes ou translucides quand la taille des particules est inférieure à 80. 10⁻³ µm.

Les compositions selon l'invention peuvent être préparées par un procédé mettant en oeuvre une première étape dans laquelle on mélange sous agitation la phase grasse comprenant le tensioactif lipophile, le tensioactif hydrophile, le composé amphiphile ionique, l'actif si présent et une deuxième étape dans laquelle on introduit la phase aqueuse dans la phase huileuse puis le mélange obtenu est maintenu sous agitation en cuve munie d'une turbine.

L'invention va être maintenant plus complètement décrite, dans ses objets et ses caractéristiques, à l'aide des exemples qui vont suivre.

### Exemples

Dans les exemples suivants, la taille des gouttes d'huiles (ou globules huileux) et la viscosité sont mesurées selon les méthodes indiquées plus haut.

### Exemple 1 : Lait pour le corps et fluide pour le visage

| | **Exemple 1 (Invention)** | **Exemple 2 (Invention)** |
|---|---|---|
| *Phase aqueuse A* | | |
| Glycerine | 5 g | 5 g |
| Conservateur | 0,65 g | 0,65 g |
| Eau déminéralisée | 24,95 g | 24,95 g |
| | | |

| *Phase huileuse B* | | |
|---|---|---|
| Potassium monocétylphosphate (Amphisol K par la société DSM) | 0,4 g | 0,4 g |
| Polysorbate 61 (Tween 61V de Croda) | 0,8 g | 0,8 g |
| Sucrose tristéarate (RYOTO SUGAR | 1,7 g | 1,7 g |
| ESTER S 370 de Mitsubishi) | | |
| Polyisobutène hydrogéné | 12,5 g | 12,5 g |
| Isopropyl Lauroyl sarcosinate | 12,5 g | 12,5 g |
| Conservateur | 0,1 g | 0,1 g |
| | | |

| *Phase aqueuse C* | | |
|---|---|---|
| Eau déminéralisée | Qsp 100 g | Qsp 100 g |
| Ammonium polyacryloyldimethyl taurate (Hostacerin AMPS de Clariant) | 0,35 g | 0,40 g |
| Acide citrique | 0,034 g | 0,034 g |
| | | |
| Viscosité (Rhéomat 180, 25°C, 200 RPM) | 0,175 Pa.s | 0,25 Pa.s |
| pH | 4,8 | 4,8 |
| Taille des gouttes à t 0 | 0,560 µm | 0,560 µm |
| Stabilité après 2 mois de 4°C à 45°C | Bonne | Bonne |

### Mode de préparation

La phase aqueuse est préparée par dissolution du conservateur et de la glycérine dans l'eau à 75°C. Le polyisobutène hydrogéné et l'isopropyl lauroyl sarcosinate sont mélangés à 75°C jusqu'à obtention d'une solution homogène ; les tensio-actifs sont alors introduits dans cette solution sous agitation. La phase aqueuse (70°C) est alors ajoutée rapidement dans la phase huileuse sous agitation à l'aide d'un mélangeur de type Maxi Lab (Olsa) à la vitesse de 4000 RPM (turbine). L'émulsification est maintenue à 75°C pendant 15 minutes à la même vitesse de rotation puis la température est diminuée à 25 °C en 20 minutes sous turbine à la vitesse de 4000 RPM.
Une émulsion huile dans eau est obtenue.
Le gélifiant ammonium polyacryloyldimethyl taurate est gonflé dans une partie de la phase aqueuse à l'aide d'une défloculeuse de type Rayneri (500 RPM) puis introduit en fin de formulation à la température ambiante sous agitation (Maxi Lab, turbine à la vitesse de 2000 RPM). L'agitation à la vitesse de 4 000 RPM est alors maintenue pendant 30 minutes.

Les émulsions de compositions 1 et 2 de l'invention sont des lait et fluide stables après deux mois de 4°C à 45°C.

### Exemples 3 à 5: Fluides visage

On prépare :
- une émulsion selon l'invention comprenant un composé amphiphile de nature ionique à un pH de 3 à 5,5 (exemple 3),
- une émulsion hors invention (exemple 4), comprenant un composé amphiphile qui n'est pas de nature ionique à un pH de 3 à 5,5 et
- une émulsion hors invention (exemple 5), comprenant de l'acide stéarique

| | **Exemple 3 (Invention)** | **Exemple 4 (Hors invention)** | **Exemple 5 (Hors invention)** |
|---|---|---|---|
| *Phase aqueuse A* | | | |
| Glycerine | 5 g | 5 g | 5 g |
| Conservateur | 0,65 g | 0,65 g | 0,65 g |
| Eau déminéralisée | 25,15 g | 25,15 g | 25,15 g |
| | | | |

| *Phase huileuse B* | | | |
|---|---|---|---|
| **Potassium monocétylphosphate** (Amphisol K par la société DSM) | **0,6 g** | **0 g** | **0 g** |
| **Sodium stearoyl glutamate** | **0 g** | **0,6 g** | **0 g** |
| **Acide stéarique** | **0 g** | **0 g** | **0,6 g** |
| Polysorbate 61 (Tween 61V de Croda) | 0,8 g | 0,8 g | 0,8 g |
| Sucrose tristéarate (RYOTO SUGAR ESTER S 370 de Mitsubishi) | 1,7 g | 1,7 g | 1,7 g |
| Polyisobutène hydrogéné | 18,5 g | 18,5 g | 18,5 g |
| Isopropyl Lauroyl sarcosinate | 6,5 g | 6,5 g | 6,5 g |
| Conservateur | 0,1 g | 0,1 g | 0,1 g |
| | | | |

| *Phase aqueuse C* | | | |
|---|---|---|---|
| Eau déminéralisée | Qsp 100 g | Qsp 100 g | Qsp 100 g |
| Ammonium polyacryloyldimethyl taurate (Hostacerin AMPS de Clariant) | 0,40 g | 0,40 g | 0,40 g |
| Acide citrique | 0,034 g | 0,034 g | 0,034 g |
| | | | |
| Viscosité (Rhéomat 180, 25°C, 200 RPM) | 0,18 Pa.s | 0,2 Pa.s | 0,15 Pa.s |
| pH | 5 | 5 | 5 |
| Taille des gouttes à t0 | 0,460 µm | 1 µm | 2 µm |
| Stabilité après 2 mois de 4°C à 45°C | Bonne | Instable | Instable |

### Mode de préparation

La phase aqueuse est préparée par dissolution du conservateur et de la glycérine dans l'eau à 75°C. Le polyisobutène hydrogéné et l'isopropyl lauroyl sarcosinate sont mélangés à 75°C jusqu'à obtention d'une solution homogène ; les tensio-actifs sont alors introduits dans cette solution sous agitation. La phase aqueuse (70°C) est alors ajoutée rapidement dans la phase huileuse sous agitation à l'aide d'un mélangeur de type Maxi Lab (Olsa) à la vitesse de 4000 RPM (turbine). L'émulsification est maintenue à 75°C pendant 15 minutes à la même vitesse de rotation puis la température est diminuée à 25 °C en 20 minutes sous turbine à la vitesse de 4000 RPM.
Une émulsion huile dans eau est obtenue.
Le gélifiant ammonium polyacryloyldimethyl taurate est gonflé dans une partie de la phase aqueuse à l'aide d'une défloculeuse de type Rayneri (500 RPM) puis introduit en fin de formulation à la température ambiante sous agitation (Maxi Lab, turbine à la vitesse de 2000 RPM). L'agitation à la vitesse de 4 000 RPM est alors maintenue pendant 30 minutes.

### Propriétés des émulsions

L'émulsion de composition 3 de l'invention est un fluide stable après deux mois de 4°C à 45°C.
Les émulsions de composition 4 et 5 (hors invention), comprenant un composé amphiphile non ionique dans la gamme 3 à 5,5 (sodium stearoyl glutamate ou de l'acide stéarique), présentent un phénomène de crémage (remontée des globules d'huile à la surface de la compostion) après 1 mois à 25°C.

Ces exemples montrent l'importance de la nature du composé amphiphile ionique sur la stabilité des émulsions.

### Exemple 6 selon l'invention : Crème de soin pour le visage

| *Phase aqueuse A* | |
|---|---|
| Glycerine | 5 g |
| Conservateur | 0,65 g |
| Eau déminéralisée | 25,15 g |
| | |

| *Phase huileuse B* | |
|---|---|
| Potassium monocétylphosphate (Amphisol K par la société DSM) | 0,6 g |
| Polysorbate 61 (Tween 61V de Croda) | 0,8 g |
| Sucrose tristéarate (RYOTO SUGAR ESTER S 370 de Mitsubishi) | 1,7 g |
| Polyisobutène hydrogéné | 18,75 g |
| Myristate d'isopropyle | 3,12 g |
| Isononanoate d'isononyle | 3,12 g |
| Conservateur | 0,1 g |
| | |

| *Phase aqueuse C* | |
|---|---|
| Eau déminéralisée | Qsp 100 g |
| Ammonium polyacryloyldimethyl taurate (Hostacerin AMPS de Clariant) | 1,5 g |
| Acide citrique | 0,034 g |
| | |
| Viscosité (Rhéomat 180, 25°C, 200 RPM) | 1,2 Pa.s |
| pH | 4,8 |
| Taille des gouttes à t0 | 0,672 µm |
| Stabilité après 2 mois de 4°C à 45°C | Bonne |

### Mode de préparation

La phase aqueuse est préparée par dissolution du conservateur et de la glycérine dans l'eau à 75°C. Le polyisobutène hydrogéné, le myristate d'isopropyle et l'isononanoate d'isononyle sont mélangés à 75°C jusqu'à obtention d'une solution homogène ; les tensio-actifs sont alors introduits dans cette solution sous agitation. La phase aqueuse (70°C) est alors ajoutée rapidement dans la phase huileuse sous agitation à l'aide d'un mélangeur de type Maxi Lab (Olsa) à la vitesse de 4000 RPM (turbine). L'émulsification est maintenue à 75°C pendant 15 minutes à la même vitesse de rotation puis la température est diminuée à 25 °C en 20 minutes sous turbine à la vitesse de 4000 RPM. Une émulsion huile dans eau est obtenue.
Le gélifiant ammonium polyacryloyldimethyl taurate est gonflé dans une partie de la phase aqueuse à l'aide d'une défloculeuse de type Rayneri (500 RPM) puis introduit en fin de formulation à la température ambiante sous agitation (Maxi Lab, turbine à la vitesse de 2000 RPM). L'agitation à la vitesse de 4 000 RPM est alors maintenue pendant 30 minutes.

### Propriétés des émulsions

L'émulsion de composition 6 de l'invention est une crème stable après deux mois de 4°C à 45°C.

## Revendications

1. Composition cosmétique ou dermatologique sous forme d'émulsion du type huile dans eau formée de globules huileux pourvus chacun d'un enrobage cristal liquide lamellaire et dispersés dans une phase aqueuse, **caractérisée en ce qu'**elle présente un pH allant de 3 à 5, et qu'elle comprend :
- au moins un agent tensioactif lipophile de HLB allant 2 à 5 choisi parmi les mono, di ou triesters de sucrose et d'acides gras comprenant de 12 à 30 atomes de carbone,
- au moins un agent tensioactif hydrophile de HLB allant 8 à 12 choisi parmi les esters d'acide gras en C12-C30 et de sorbitol et/ou de sorbitan oxyéthylénés comportant de 2 à 30 groupes OE,
- au moins un composé amphiphile de nature ionique à un pH allant de 3 à 5 choisi parmi les sels de sodium et de potassium du monocétyle phosphate.

2. Composition selon la revendication 1, **caractérisée en ce que** l'agent tensioactif lipophile est choisi parmi les mono, di ou triesters de sucrose et d'acides gras comprenant de 14 à 20 atomes de carbone tel que l'acide stéarique, en particulier le tristérarate de sucrose, le distéarate de sucrose et leurs mélanges,

3. Composition selon l'une des revendications précédentes, **caractérisée en ce que** l'agent tensioactif lipophile présentant un HLB compris entre 2 et 5 représente de 0,1 à 15% en poids par rapport au poids total de la composition, de préférence de 0,5 à 5% en poids.

4. Composition selon l'une des revendications précédentes, **caractérisée en ce que** l'agent tensioactif hydrophile présentant un HLB allant de 8 à 12 représente de 0,05 à 15% en poids par rapport au poids total de la composition, de préférence de 0,1 à 10% en poids et de préférence de 0,5 à 5% en poids.

5. Composition selon l'une des revendications précédentes, **caractérisée en ce que** l'agent tensioactif hydrophile présentant un HLB allant de 8 à 12 est choisi parmi les esters d'acide stéarique et de sorbitol et/ou de sorbitan comprenant de 2 à 20 groupes OE.

6. Composition selon l'une des revendications précédentes, **caractérisée en ce que** l'agent tensioactif hydrophile présentant un HLB allant de 8 à 12 est choisi parmi le monostéarate de sorbitan polyoxyéthyléné 20 OE, le monostéarate de sorbitan polyoxyéthyléné 4 OE, le sorbeth 3 isostéarate, le tristéarate de sorbitan polyoxyéthyléné 20 OE.

7. Composition selon l'une quelconque des revendications précédentes, **caractérisée en ce que** le composé amphiphile ionique représente de 0,05 à 10% en poids par rapport au poids total de la composition, de préférence de 0,5 à 5% en poids.

8. Compositions selon l'une quelconque des revendications précédentes, **caractérisée en ce que** les quantités relatives de tensioactifs lipophile, hydrophile et de composé amphiphile ionique vont de préférence respectivement de 35 à 55% en poids, de 25 à 40% en poids et de 15 à 35% en poids par rapport au poids total de l'ensemble des tensioactifs lipophile, hydrophile et du composé amphiphile ionique.

9. Composition selon l'une quelconque des revendications précédentes, **caractérisée en ce que** la phase huileuse comprend au moins une huile ester choisie parmi les dérivés lipophiles d'acides aminés, tels que le lauroyl sarcosinate d'isopropyle, les esters d'acide gras, linéaires ou ramifiés, comprenant de 8 à 29 atomes de carbone et d'alcools, linéaires ou ramifiés, de préférence ramifié, comprenant de 3 à 15 atomes de carbone, tels que le myristate d'ispropropyle, l'isononanoate d'isononyle et leurs mélanges.

10. Composition selon l'une quelconque des revendications précédentes, **caractérisée en ce que** la phase huileuse comprend au moins 25% en poids d'au moins une huile ester choisie parmi les dérivés lipophiles d'acides aminés, tels que le lauroyl sarcosinate d'isopropyle, les esters d'acide gras, linéaires ou ramifiés, comprenant de 8 à 29 atomes de carbone et d'alcools, linéaires ou ramifiés, comprenant de 3 à 15 atomes de carbone, tels que le myristate d'ispropropyle, l'isononanoate d'isononyle et leurs mélanges.

11. Composition selon l'une quelconque des revendications précédentes, **caractérisée en ce que** qu'elle comprend un gélifiant hydrophile.

12. Composition selon l'une quelconque des revendications précédentes, **caractérisée en ce que** qu'elle présente une viscosité supérieure ou égale à 0,1 Pa.s, pouvant aller par exemple de 0,1 Pa.s à 100 Pa.s, de préférence de 0,1 Pa.s à 10 Pa.s à une température de 25°C.

13. Composition selon l'une quelconque des revendications précédentes, **caractérisée en ce que** qu'elle présente un pH allant de 4 à 5.

14. Procédé de traitement cosmétique de la peau, des cheveux, et/ou des lèvres, **caractérisé en ce qu'**on applique sur la peau, les cheveux et/ou les lèvres, une composition cosmétique selon l'une quelconque des revendications 1 à 13.

## Patentansprüche

1. Kosmetische oder dermatologische Zusammensetzung in Form einer Öl-in-Wasser-Emulsion aus Ölkügelchen, die jeweils mit einer lamellaren Flüssigkristallumhüllung versehen und in einer wässrigen Phase dispergiert sind, **dadurch gekennzeichnet, dass** sie einen pH-Wert im Bereich von 3 bis 5 aufweist und dass sie Folgendes umfasst:
- mindestens ein lipophiles Tensid mit einem HLB-Wert im Bereich von 2 bis 5, das aus Mono-, Di-oder Triestern von Saccharose und Fettsäuren mit 12 bis 30 Kohlenstoffatomen ausgewählt ist,
- mindestens ein hydrophiles Tensid mit einem HLB-Wert im Bereich von 8 bis 12, das aus oxyethylenierten Estern von C12-C30-Fettsäure und Sorbitol und/oder Sorbitan mit 2 bis 30 EO-Gruppen ausgewählt ist,
- mindestens eine amphiphile Verbindung mit ionischer Beschaffenheit bei einem pH-Wert im Bereich von 3 bis 5, die aus Natrium- und Kaliumsalzen von Monocetylphosphat ausgewählt ist.

2. Zusammensetzung nach Anspruch 1, **dadurch gekennzeichnet, dass** das lipophile Tensid aus Mono-, Di-oder Triestern von Saccharose und Fettsäuren mit 14 bis 20 Kohlenstoffatomen wie Stearinsäure, insbesondere Saccharosetristearat und Saccharosedistearat, und Mischungen davon ausgewählt ist.

3. Zusammensetzung nach einem der vorhergehenden Ansprüche, **dadurch gekennzeichnet, dass** das lipophile Tensid mit einem HLB-Wert zwischen 2 und 5 0,1 bis 15 Gew.-%, bezogen auf das Gesamtgewicht der Zusammensetzung, vorzugsweise 0,5 bis 5 Gew.-%, ausmacht.

4. Zusammensetzung nach einem der vorhergehenden Ansprüche, **dadurch gekennzeichnet, dass** das hydrophile Tensid mit einem HLB-Wert im Bereich von 8 bis 12 0,05 bis 15 Gew.-%, bezogen auf das Gesamtgewicht der Zusammensetzung, vorzugsweise 0,1 bis 10 Gew.-% und vorzugsweise 0,5 bis 5 Gew.-% ausmacht.

5. Zusammensetzung nach einem der vorhergehenden Ansprüche, **dadurch gekennzeichnet, dass** das hydrophile Tensid mit einem HLB-Wert im Bereich von 8 bis 12 aus Estern von Stearinsäure und Sorbitol und/oder Sorbitan mit 2 bis 20 EO-Gruppen ausgewählt ist.

6. Zusammensetzung nach einem der vorhergehenden Ansprüche, **dadurch gekennzeichnet, dass** das hydrophile Tensid mit einem HLB-Wert im Bereich von 8 bis 12 aus mit 20 EO polyoxyethyleniertem Sorbitanmonostearat, mit 4 EO polyoxyethyleniertem Sorbitanmonostearat, Sorbeth-3-isostearat und mit 20 EO polyoxyethyleniertem Sorbitantristearat ausgewählt ist.

7. Zusammensetzung nach einem der vorhergehenden Ansprüche, **dadurch gekennzeichnet, dass** die ionische amphiphile Verbindung 0,05 bis 10 Gew.-%, bezogen auf das Gesamtgewicht der Zusammensetzung, vorzugsweise 0,5 bis 5 Gew.-%, ausmacht.

8. Zusammensetzung nach einem der vorhergehenden Ansprüche, **dadurch gekennzeichnet, dass** die relativen Mengen von lipophilen und hydrophilen Tensiden und ionischer amphiphiler Verbindung vorzugsweise 35 bis 55 Gew.-%, 25 bis 40 Gew.-% bzw. 15 bis 35 Gew.-%, bezogen auf das Gesamtgewicht der Gesamtheit der lipophilen und hydrophilen Tenside und der ionischen amphiphilen Verbindung, betragen.

9. Zusammensetzung nach einem der vorhergehenden Ansprüche, **dadurch gekennzeichnet, dass** die ölige Phase mindestens ein Esteröl, das aus lipophilen Derivaten von Aminosäuren, wie Isopropyllauroylsarcosinat, Estern von linearen oder verzweigten Fettsäuren mit 8 bis 29 Kohlenstoffatomen und linearen oder verzweigten, vorzugsweise verzweigten, Alkoholen mit 3 bis 15 Kohlenstoffatomen, wie Isopropylmyristat und Isononylisononanoat, und Mischungen davon ausgewählt ist, umfasst.

10. Zusammensetzung nach einem der vorhergehenden Ansprüche, **dadurch gekennzeichnet, dass** die ölige Phase mindestens 25 Gew.-% mindestens eines Esteröls, das aus lipophilen Derivaten von Aminosäuren, wie Isopropyllauroylsarcosinat, Estern von linearen oder verzweigten Fettsäuren mit 8 bis 29 Kohlenstoffatomen und linearen oder verzweigten Alkoholen mit 3 bis 15 Kohlenstoffatomen, wie Isopropylmyristat und Isononylisononanoat, und Mischungen davon ausgewählt ist, umfasst.

11. Zusammensetzung nach einem der vorhergehenden Ansprüche, **dadurch gekennzeichnet, dass** sie einen hydrophilen Gelbildner umfasst.

12. Zusammensetzung nach einem der vorhergehenden Ansprüche, **dadurch gekennzeichnet, dass** sie eine Viskosität größer gleich 0,1 Pa.s, die beispielsweise im Bereich von 0,1 Pa.s bis 100 Pa.s und vorzugsweise von 0,1 Pa.s bis 10 Pa.s liegen kann, bei einer Temperatur von 25°C aufweist.

13. Zusammensetzung nach einem der vorhergehenden Ansprüche, **dadurch gekennzeichnet, dass** sie einen pH-Wert im Bereich von 4 bis 5 aufweist.

14. Verfahren zur kosmetischen Behandlung der Haut, der Haare und/oder der Lippen, **dadurch gekennzeichnet, dass** man auf die Haut, die Haare und/oder die Lippen eine kosmetische Zusammensetzung nach einem der Ansprüche 1 bis 13 aufbringt.

## Claims

1. Cosmetic or dermatological composition in the form of an oil-in-water emulsion formed from oil globules which each have a lamellar liquid crystal coating and which are dispersed in an aqueous phase, **characterized in that** it has a pH ranging from 3 to 5, and that it comprises:
- at least one lipophilic surfactant having an HLB ranging from 2 to 5 chosen from monoesters, diesters or triesters of sucrose and of fatty acids comprising from 12 to 30 carbon atoms,
- at least one hydrophilic surfactant having an HLB ranging from 8 to 12 chosen from oxyethylenated esters of a C₁₂-C₃₀ fatty acid and of sorbitol and/or of sorbitan comprising from 2 to 30 OE groups,
- at least one amphiphilic compound of ionic nature at a pH ranging from 3 to 5 chosen from potassium and sodium salts of monocetyl phosphate.

2. Composition according to Claim 1, **characterized in that** the lipophilic surfactant is chosen from monoesters, diesters or triesters of sucrose and of fatty acids comprising from 14 to 20 carbon atoms such as stearic acid, in particular sucrose tristearate, sucrose distearate and mixtures thereof.

3. Composition according to either of the preceding claims, **characterized in that** the lipophilic surfactant having an HLB between 2 and 5 represents from 0.1% to 15% by weight relative to the total weight of the composition, preferably from 0.5% to 5% by weight.

4. Composition according to one of the preceding claims, **characterized in that** the hydrophilic surfactant having an HLB ranging from 8 to 12 represents from 0.05% to 15% by weight relative to the total weight of the composition, preferably from 0.1% to 10% by weight and preferably from 0.5% to 5% by weight.

5. Composition according to one of the preceding claims, **characterized in that** the hydrophilic surfactant having an HLB ranging from 8 to 12 is chosen from esters of stearic acid and of sorbitol and/or of sorbitan comprising from 2 to 20 OE groups.

6. Composition according to one of the preceding claims, **characterized in that** the hydrophilic surfactant having an HLB ranging from 8 to 12 is chosen from polyoxyethylenated 20 OE sorbitan monostearate, polyoxyethylenated 4 OE sorbitan monostearate, sorbeth-3 isostearate, and polyoxyethylenated 20 OE sorbitan tristearate.

7. Composition according to any one of the preceding claims, **characterized in that** the ionic amphiphilic compound represents from 0.05% to 10% by weight relative to the total weight of the composition, preferably from 0.5% to 5% by weight.

8. Composition according to any one of the preceding claims, **characterized in that** the relative amounts of lipophilic and hydrophilic surfactants and of ionic amphiphilic compound preferably range respectively from 35% to 55% by weight, from 25% to 40% by weight and from 15% to 35% by weight relative to the total weight of all of the lipophilic and hydrophilic surfactants and of the ionic amphiphilic compound.

9. Composition according to any one of the preceding claims, **characterized in that** the oily phase comprises at least one ester oil chosen from lipophilic derivatives of amino acids, such as isopropyl lauroyl sarcosinate, esters of linear or branched fatty acids comprising from 8 to 29 carbon atoms and of linear or branched, preferably branched, alcohols comprising from 3 to 15 carbon atoms, such as isopropyl myristate, isononyl isononanoate and mixtures thereof.

10. Composition according to any one of the preceding claims, **characterized in that** the oily phase comprises at least 25% by weight of at least one ester oil chosen from lipophilic derivatives of amino acids, such as isopropyl lauroyl sarcosinate, esters of linear or branched fatty acids comprising from 8 to 29 carbon atoms and of linear or branched alcohols comprising from 3 to 15 carbon atoms, such as isopropyl myristate, isononyl isononanoate and mixtures thereof.

11. Composition according to any one of the preceding claims, **characterized in that** it comprises a hydrophilic gelling agent.

12. Composition according to any one of the preceding claims, **characterized in that** it has a viscosity greater than or equal to 0.1 Pa.s, which may range for example from 0.1 Pa.s to 100 Pa.s, preferably from 0.1 Pa.s to 10 Pa.s, at a temperature of 25°C.

13. Composition according to any one of the preceding claims, **characterized in that** it has a pH ranging from 4 to 5.

14. Cosmetic treatment process for the skin, the hair and/or the lips, **characterized in that** a cosmetic composition according to any one of Claims 1 to 13 is applied to the skin, the hair and/or the lips.
